# EUROPEAN PATENT APPLICATION

(11) **EP 1 528 053 A1**
(43) Date of publication of application: **04.05.2005**
(21) Application number: 03024865.2
(22) Date of filing: 31.10.2003
(51) Int. Cl.: C07C 67/31, C07C 69/675

(54) **Process for the preparation of (S)- or (R)-4-halo-3-hydroxybutyrates**

(71) Applicant: Lonza AG, 4052 Basel (CH)
(72) Inventor: The designation of the inventor has not yet been filed

(57) **Abstract**

Process for the preparation of enantiomerically pure (*S*)- or (*R*)-4-halo-3-hydroxybutyrates of formula 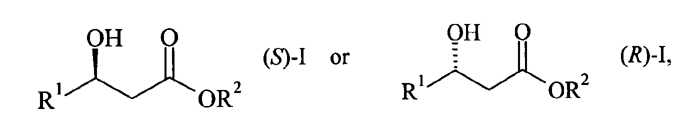 wherein R¹ is CH₂X, CHX₂ or CX₃ and X independently represents Cl and/or Br and wherein R² is C₁₋₆-alkyl, C₃₋₈-cycloalkyl, aryl or aralkyl, each aryl or aralkyl being optionally further substituted with one or more C₁₋₄-alkyl groups and/or halogen atoms, by asymmetric hydrogenation of 4-halo-3-oxobutyric acid esters of formula 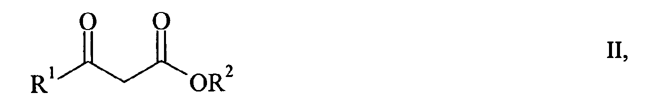 wherein R¹, R² and X are as defined above
in the presence of a catalyst of a ruthenium complex comprising a chiral ligand of the formula

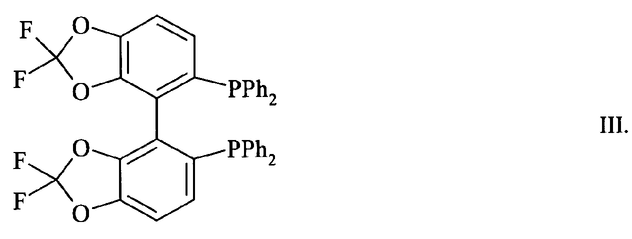

## Description

The invention relates to a process for the preparation of enantiomerically pure (*S*)- or (*R*)-4-halo-3-hydroxybutyrates of formula 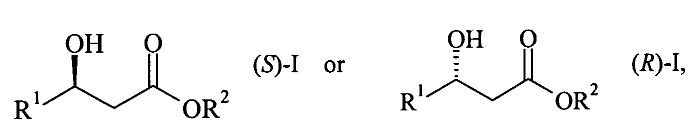 wherein R¹ is CH₂X, CHX₂ or CX₃ and X independently represents Cl and/or Br and wherein R² is C₁₋₆-alkyl, C₃₋₈-cycloalkyl, aryl or aralkyl by asymmetric hydrogenation of 4-halo-3-oxobutyrates of formula 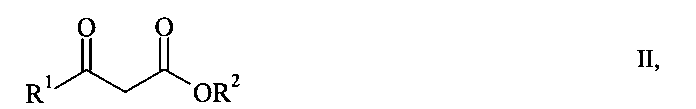 wherein R¹, R² and X are as defined above.

Here and hereinbelow the term "enantiomerically pure compound" comprises optically active compounds with an enantiomeric excess (ee) of at least 90 %.

Here and hereinbelow the term "C₁₋₆-alkyl" represents a linear or branched alkyl group having 1 to 6 carbon atoms, for example methyl, ethyl, propyl, isopropyl, *n*-butyl, isobutyl, *sec*-butyl, *tert*-butyl, pentyl and hexyl, which can be optionally further substituted by one or more halogen atoms.

Here and hereinbelow the term "C₁₋₄-alkoxy" represents a linear or branched alkoxy group having 1 to 6 carbon atoms, for example methoxy, ethoxy, propyloxy, isopropyloxy, *n*-butyloxy, isobutyloxy, *sec*-butyloxy, *tert*-butyloxy, which can be optionally further substituted by one or more halogen atoms.

Here and hereinbelow the term "C₃₋₈-cycloalkyl" represents a cycloaliphatic group having 3 to 8 carbon atoms, i.e. cyclopropyl, cyclobutyl, cyclopentyl, cyclohexyl, cycloheptyl and cyclooctyl.

Here and hereinbelow the term "aryl" represents an aromatic group, preferably phenyl or naphthyl, which is , optionally substituted with one or more halogen atoms, C₁₋₄-alkyl and/or C₁₋₄-alkoxy, wherein the alkyl and alkoxy substituents optionally are further substituted with one or more halogen atoms.

Here and hereinbelow the term "aralkyl" represents a linear C₁₋₄-alkyl moiety optionally further substituted by halogen atoms, which is connected to an optionally further substituted aryl moiety as defined above.

Generally 3-hydroxybutyrates and derivatives thereof are useful key intermediates in preparative industrial chemistry. Among the derivatives especially ethyl (*S*)-4-chloro-3-hydroxybutyrate is an important building block for the preparation ofHMG-CoA reductase inhibitors like Rosuvastatin® or Atorvastatin®. To provide said hydroxybutyrates in pharmaceutically acceptable qualities and to avoid costly separation of the enantiomers the processes for asymmetric hydrogenation should result in high ee, preferably in the range of 90 to 100 %.

Asymmetric hydrogenation is mostly carried out with chiral transition metal-ligand complexes, wherein the transition metal is ruthenium or rhodium and wherein the ligands are often substituted chiral bidentate diphosphines.

WO-A-02/40492 discloses asymmetric hydrogenation of ethyl 4-chloro-3-oxobutyrate using a catalyst containing a (*S*)-6-methoxy-5,6'-benzo-2,2'-bis(diphenylphosphino)-biphenyl ligand. The (*S*)-product is obtained with an ee of 83%.

A new class of methylenedioxo and ethylenedioxo substituted diphosphine ligands and their use for the asymmetric hydrogenation of methyl 2-benzamidomethyl-3-oxobutyrate has been described in EP-A-0 850 945. Pai, C.-C. et al, *Tetrahedron Lett.* **2002,** *43,* 2789-2792 discloses the use of said ligands for the asymmetric hydrogenation of ethyl 4-chloro-3-oxobutyrate.

EP-A-0 955 303 describes the asymmetric hydrogenation of 4-methylene-2-oxetanone (diketene) to 4-methyl-2-oxetanone, a β-lactone of 3-hydroxybutyric acid using ruthenium iodo derivatives of the ligands disclosed in EP-A-0 850 945.

The technical problem to be solved by the present invention was to provide an alternative general process for the asymmetric hydrogenation of 4-halo-3-oxobutyrates to yield enantiomerically pure (*S*)- or (*R*)-4-halo-3-hydroxybutyrates.

The problem could be solved according to the process of claim 1.

The invention provides a process for the preparation of enantiomerically pure (*S*)- or (*R*)-4-halo-3-hydroxybutyric acid esters of formula 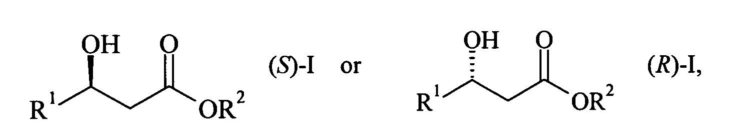 wherein R¹ is CH₂X, CHX₂ or CX₃ and X independently represents Cl and/or Br and wherein R² is C₁₋₆-alkyl, C₃₋₈-cycloalkyl, aryl or aralkyl, each aryl or aralkyl being optionally further substituted with one or more C₁₋₄-alkyl groups and/or halogen atoms, by asymmetric hydrogenation of 4-halo-3-oxobutyric acid esters of formula 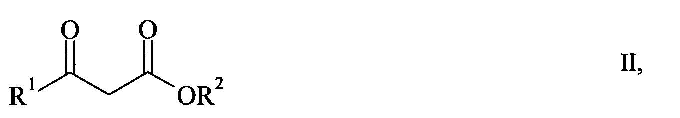 wherein R¹, R² and X are as defined above
in the presence of a catalyst of a ruthenium complex comprising a chiral ligand of formula

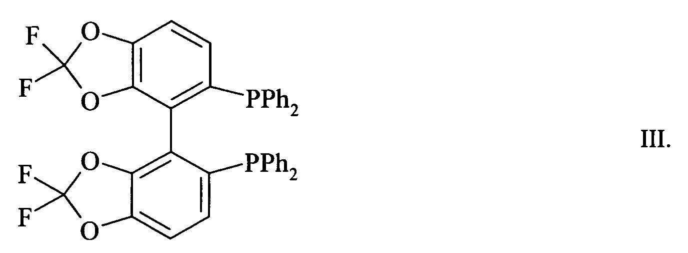

In a preferred embodiment R¹ is CH₂X and X represents Cl or Br, more preferably R¹ is CH₂X and X represents Cl.

WO 03/029259 discloses a process for the asymmetric hydrogenation of alkyl and aryl derivatives of 3-oxoacid esters using a ruthenium complex of the ligand of formula III.

Several examples for asymmetric hydrogenation of esters of fluorinated 3-oxoacids are presented, but hydrogenations of compounds of formula II are not disclosed. All fluorinated aliphatic 3-hydroxyacid esters disclosed therein, like ethyl 4,4,4-trifluoro-3-hydroxybutyrate, are obtainable only at moderate ee-levels in the range of 70 to 80%.

Regarding EP-A-0 850 945 in the light of the experimental results of WO 03/029259 it seemed very unlikely that transition metal complexes of 5,5'-bis(diphenylphosphanyl)-2,2,2',2'-tetrafluoro-4,4'-bi[benzo-1,3-dioxolyl] ligands of formula III (Fluoxphos) would be suitable for the asymmetric hydrogenation of compounds of formula II.

Surprisingly the asymmetric hydrogenation of 4-chloro-3-oxobutyrates and 4,4,4-trichloro-3-oxobutyrates using ruthenium complexes containing the ligand of formula III ((-)-Fluoxphos and (+)-Fluoxphos) gives very high ee-levels.

In a preferred embodiment R² is selected from the group consisting of methyl, ethyl, propyl, isopropyl, *n*-butyl and *tert*-butyl. More preferred R² is methyl, ethyl or isopropyl. Particularly preferred R² is methyl or ethyl.

In another preferred embodiment R² is, optionally substituted, phenyl or naphthyl.

The hydrogenation is carried out with a catalyst solution in a polar solvent like C₁₋₄-alcohols, dimethylsulfoxide (DMSO), dimethylformamide (DMF), acetonitrile (MeCN) or mixtures thereof. Preferably the polar solvent is methanol, ethanol or isopropyl alcohol or a mixture thereof. The solution may contain further solvent additives like dichloromethane.

The catalyst solution can be prepared in situ by dissolving a ruthenium salt Ruⁿ⁺Y⁻ₙ, wherein n is 2 or 3 and wherein Y⁻ is Cl⁻, Br⁻, I⁻, BF₄⁻, AsF₆⁻, SbF₆⁻, PF₆⁻, ClO₄⁻ or OTf⁻ (trifluormethane sulfonate or triflate) or another suitable counterion in a polar solvent and mixing with a suitable amount of the ligand of formula III, optionally further mixed with at least one stabilizing ligand.

Alternatively, the catalyst solution can be obtained by mixing a catalyst precursor complex, i.e. a preformed ruthenium complex which already contains at least one stabilizing ligand, in a polar solvent with a suitable amount of further ligands. The catalyst precursor complex comprises at least one stabilizing ligand such as a diene, alkene or arene. In a preferred embodiment the stabilizing ligand is 1,5-cyclooctadiene (cod), norbornadiene (nbd) or *p*-cymene (cym). Particularly preferred the stabilizing ligand is *p*-cymene.
In a further preferred embodiment the catalyst precursor complex comprises at least one chiral ligand of formula III (Fluoxphos).
In a further particularly preferred embodiment the catalyst precursor complex comprises at least one polar solvent molecule as stabilizing ligand, such as dimethylsulfoxide (DMSO), dimethylformamide (DMF) or acetonitrile (MeCN).
Examples for catalyst precursor complexes containing such stabilizing ligands are [Ru₂Cl₄(cym)₂], [Ru₂Br₄(cym)₂], [RuCl(Fluoxphos)(benzene)]Cl, [RuCl₂(Fluoxphos)·DMF], [RuCl₂(Fluoxphos)·DMSO] and [Ru₂Cl₄(cod)₂·MeCN].

Furthermore, the catalyst solution can be obtained by dissolving a preformed chiral ruthenium complex which already contains all required ligands.

Several examples for general applicable methods for the preparations of catalysts and catalyst solutions are disclosed in Ashworth, T. V. et al. *S*. *Afr*. *J*. *Chem*. **1987**, *40*, 183-188, WO 00/29370 and Mashima, K. *J*. *Org*. *Chem*. **1994,** *59*, 3064-3076.

In a preferred embodiment the catalyst precursor complex is prepared by mixing bis[(1-isopropyl-4-methylbenzene)dichloro ruthenium] of the formula [Ru₂Cl₄(cym)₂] with either the (-)-Fluoxphos or the (+)-Fluoxphos ligand in a polar solvent.

Preferably the metal salt Ruⁿ⁺Y⁻ₙ, or the ruthenium complex is mixed with the chiral bidentate phosphine at a ratio of 1:5 to 5:1. More preferably the Ru/phosphine ratio is in the range of 1:2 to 2:1. Most preferably the Ru/phosphine ratio is 1:1.

In a particular embodiment the counterion of the transition metal salt, the catalytic precursor complex and the ruthenium complex of the ligand of formula III is Cl⁻, Br⁻, I⁻, BF₄⁻, AsF₆⁻, SbF₆⁻, PF₆⁻, ClO₄⁻ or OTf⁻, more preferably Cl⁻, I⁻ or BF₄⁻.

In a preferred embodiment the hydrogen pressure during the reaction is in the range of 1 to 60 bar and more particularly preferred in the range of 2 to 35 bar.

The hydrogenation can be carried out at a temperature in the range of 20 to 150 °C. Preferably the temperature is in the range of 70 to 130 °C. Particularly preferred the temperature is in the range of 80 to 120 °C.

The present invention is illustrated by the following non-limiting examples.

### Examples:

### Example 1:

Ethyl (*S*)-4-chloro-3-hydroxybutyrate (Formula (*S*)-I, R¹ = CH₂Cl, R² = ethyl):
In a 150 mL autoclave under argon atmosphere RuCl₃ (10.2 mg, 0.049 mmol), (-)-5,5'-bis(diphenylphosphanyl)-2,2,2',2'-tetrafluoro-4,4'-bi[benzo-1,3-dioxolyl] (i.e. (-)-Fluoxphos) (34.2 mg, 0.050 mmol) and ethyl 4-chloro-3-oxobutyrate (0.81 g, 4.9 mmol, approx. 98.5%) is dissolved in degassed ethanol (30 mL). After flushing the autoclave with argon hydrogenation is carried out during 2 hours at 80 °C and at 4 bar hydrogen pressure. After cooling to room temperature the reaction solution is directly analyzed by GC for conversion (column: HP-101 25 m / 0.2 mm) and enantiomeric excess (ee) (column: Lipodex-E 25 m / 0.25 mm). Conversion is 100% at an ee of 97.6%.

### Example 2:

Ethyl (*S*)-4-chloro-3-hydroxybutyrate (Formula (*S*)-I, R¹ = CH₂Cl, R² = ethyl):
In a 250 mL autoclave in an argon atmosphere bis[(1-isopropyl-4-methylbenzene)dichloro ruthenium] (i.e. [Ru₂Cl₄(cym)₂]) (6.7 mg, 0.011 mmol), (-)-Fluoxphos (16.0 mg, 0.023 mmol) and ethyl 4-chloro-3-oxobutyrate (9.11 g, 54.5 mmol, approx. 98.5%) is dissolved in degassed ethanol (30 mL). After flushing the autoclave with argon hydrogenation is carried out during 2.3 hours at 90 °C and at 30 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 95.8%.

### Example 3:

Ethyl (*R*)-4-chloro-3-hydroxybutyrate (Formula (*R*)-I, R¹ = CH₂Cl, R² = ethyl):
In a 1 L autoclave under argon atmosphere RuCl₃ (10.0 mg, 0.048 mmol), (+)-Fluoxphos (36.1 mg, 0.053 mmol) and ethyl 4-chloro-3-oxobutyrate (8.0 g, 48 mmol, approx. 98.5%) is dissolved in degassed ethanol (170 mL) and dichloromethane (40 mL). After flushing the autoclave with argon hydrogenation is carried out during 90 minutes at 82 °C and at 35 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 97.8%.

### Example 4:

Ethyl (*R*)-4-chloro-3-hydroxybutyrate (Formula (*R*)-I, R¹ = CH₂Cl, R² = ethyl):
In a 1 L autoclave under argon atmosphere [Ru₂Cl₄(cod)₂·MeCN] (15.8 mg, 0.026 mmol, prepared from RuCl₃ and 1,5-cyclooctadiene as disclosed in Ashworth, T. V. et al. *S. Afr*. *J*. *Chem*. **1987**, *40*, 183-188, (+)-Fluoxphos (36.1 mg, 0.053 mmol) and ethyl 4-chloro-3-oxobutyrate (8.0 g, 48 mmol, approx. 98.5%) is dissolved in degassed ethanol (170 mL) and dichloromethane (40 mL). After flushing the autoclave with argon hydrogenation is carried out during 80 minutes at 82 °C and at 35 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 97.3%.

### Example 5:

Ethyl (*S*)-4-chloro-3-hydroxybutyrate (Formula (*S*)-I, R¹ = CH₂Cl, R² = ethyl):
In a 1 L autoclave under argon atmosphere [Ru₂Cl₄(cym)₂] (5.0 mg, 0.008 mmol, (-)-Fluoxphos (12.1 mg, 0.018 mmol) and ethyl 4-chloro-3-oxobutyrate (27.0 g, 162 mmol, approx. 98.5%) is dissolved in degassed ethanol (340 mL) and dichloromethane (80 mL). After flushing the autoclave with argon hydrogenation is carried out during 70 minutes at 110 °C and at 22 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 98.1%.

### Example 6:

Ethyl (*S*)-4-chloro-3-hydroxybutyrate (Formula (*S*)-I, R¹ = CH₂Cl, R² = ethyl):
In a 1 L autoclave under argon atmosphere [RuI((-)-Fluoxphos)(cym)]I (35 mg, 0.030 mmol, prepared from (-)-Fluoxphos and [Ru₂I₄(cym)₂] as disclosed in WO 00/29370, and ethyl 4-chloro-3-oxobutyrate (33.4 g, 200 mmol, approx. 98.5%) is dissolved in degassed ethanol (340 mL) and dichloromethane (80 mL). After flushing the autoclave with argon hydrogenation is carried out during 170 minutes at 100 °C and at 22 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 97.4%.

### Example 7:

Ethyl (*S*)-4-chloro-3-hydroxybutyrate (Formula (*S*)-I, R¹ = CH₂Cl, R² = ethyl):
In a 1 L autoclave under argon atmosphere [RuCl((-)-Fluoxphos)(cym)]BF₄ (23 mg, 0.022 mmol, prepared from (-)-Fluoxphos and [Ru₂Cl₄(cym)₂] and AgBF₄ as disclosed in Mashima, K., *J*. *Org*. *Chem*. **1994**, *59*, 3064-3076, and ethyl 4-chloro-3-oxobutyrate (33.4 g, 200 mmol, approx. 98.5%) is dissolved in degassed ethanol (340 mL) and dichloromethane (80 mL). After flushing the autoclave with argon hydrogenation is carried out during 180 minutes at 100 °C and at 22 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 98.0%.

### Example 8:

*tert*-Butyl 4-chloro-3-hydroxybutyrate (Formula I, R¹ = CH₂Cl, R² = *tert*-butyl):
In a 150 mL autoclave under argon atmosphere [Ru₂Cl₄(cym)₂] (7.7 mg, 0.013 mmol), (+)-Fluoxphos (17.9 mg, 0.026 mmol) and *tert*-butyl 4-chloro-3-oxobutyrate (assay 96.9% by GC, 1.0 g, 5.2 mmol) is dissolved in degassed ethanol (30 mL). After flushing the autoclave with argon hydrogenation is carried out during 4 hours at 80 °C and at 4 bar hydrogen pressure. After cooling to room temperature the reaction solution is directly analyzed by GC for conversion (column: HP-101 25 m / 0.2 mm). Analysis for enantiomeric excess is performed by ¹H-NMR using europium(III) tris[3-(trifluoromethyl-hydroxymethylene)-(+)-camphorate] as shift reagent and CDCl₃ as solvent. Conversion is >99%. Yield is approx. 67% at an ee of one enantiomer of 93.2%.

### Example 9:

Ethyl 4,4,4-trichloro-3-hydroxybutyrate (Formula I, R¹ = CCl₃, R² = ethyl):
In a 150 mL autoclave under argon atmosphere [Ru₂Cl₄(cym)₂] (7.6 mg, 0.012 mmol), (+)-Fluoxphos (17.8 mg, 0.026 mmol) and ethyl 4,4,4-trichloro-3-oxobutyrate (assay 97%, 1.21 g, 5.2 mmol) is dissolved in degassed ethanol (30 mL). After flushing the autoclave with argon hydrogenation is carried out during 7.5 hours at 80 °C and at 4 bar hydrogen pressure. After cooling to room temperature the reaction solution is directly analyzed by GC for conversion (column: HP-101 25 m / 0.2 mm). Conversion is 99% at an ee of one enantiomer of 95.3%. Analysis for enantiomeric excess is performed by ¹H-NMR using europium(III) tris[3-(trifluoromethylhydroxymethylene)-(+)-camphorate] as shift reagent and CDCl₃ as solvent.

### Comparative Example 1:

Ethyl (*S*)-4-chloro-3-hydroxybutyrate (Formula (*S*)-I, R¹ = CH₂Cl, R² = ethyl):
In a 250 mL autoclave in an argon atmosphere [Ru₂Cl₄(cym)₂] (6.7 mg, 0.011 mmol), (-)-5,5'-bis(diphenylphosphanyl)-4,4'-bi[benzo-1,3-dioxolyl] (14.3 mg, 0.023 mmol) and ethyl 4-chloro-3-oxobutyrate (9.10 g, 54.5 mmol, approx. 98.5%) is dissolved in degassed ethanol (30 mL). After flushing the autoclave with argon hydrogenation is carried out during 2.3 hours at 90 °C and at 30 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 89.5%.

## Claims

1. Process for the preparation of enantiomerically pure (*S*)- or (*R*)-4-halo-3-hydroxybutyrates of formula 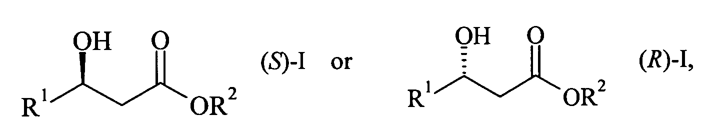 wherein R¹ is CH₂X, CHX₂ or CX₃ and X independently represents Cl and/or Br and wherein R² is C₁₋₆-alkyl, C₃₋₆-cycloalkyl, aryl or aralkyl, each aryl or aralkyl being optionally further substituted with one or more C₁₋₄-alkyl groups and/or halogen atoms,
which process comprises the asymmetric hydrogenation of 4-halo-3-oxobutyates of formula 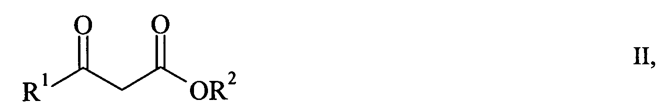 wherein R¹, R² and X are as defined above
in the presence of a catalyst of a ruthenium complex comprising a chiral ligand of formula

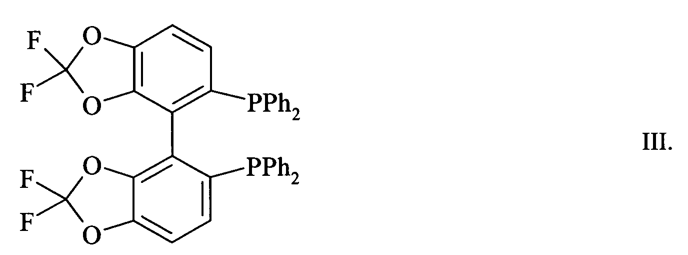

2. The process of claim 1, wherein the ruthenium complex comprising aligand of formula III comprises at least one diene, alkene or arene or polar solvent molecule as stabilizing ligand.

3. The process of claim 1 or 2, wherein the ruthenium complex comprising a ligand of formula III comprises at least one molecule of 1,5-cyclooctadiene or *p*-cymene as stabilizing ligand.

4. The process of one of claims 1 to 3, wherein the hydrogenation is carried out in a solution comprising a polar solvent selected from the group consisting of C₁₋₄-alcohols, dimethylsulfoxide, dimethylformamide, acetonitrile and mixtures thereof, wherein the solvent optionally contains further solvent additives.

5. The process of any one of claims 1 to 4, wherein the counterion of the ruthenium complex is selected from the group consisting of Cl⁻, Br⁻, I⁻, BF₄⁻, AsF₆⁻, SbF₆⁻, PF₆⁻, ClO₄⁻ and OTf⁻.

6. The process of any one of claims 1 to 5, wherein the ruthenium complex is prepared by mixing the complex of formula [Ru₂Cl₄(cym)₂] with the Fluoxphos ligand in a polar solvent.

7. The process of any of claims 1 to 6, wherein the hydrogen pressure during the reaction is in the range of 1 to 60 bar and preferably in the range of 2 to 35 bar.
